# EUROPEAN PATENT APPLICATION

(11) **EP 4 410 988 A1**
(43) Date of publication of application: **07.08.2024**
(21) Application number: 23154267.1
(22) Date of filing: 31.01.2023
(51) Int. Cl.: C12N 15/864, A61K 48/00, C12N 15/10, C40B 40/02, C07K 14/015, C12N 15/35

(54) **AN AAV2-VECTOR VARIANT FOR TARGETED TRANSFER OF GENES**

(71) Applicant: Universitätsklinikum Hamburg-Eppendorf, 20246 Hamburg (DE)
(72) Inventor: HUBER, Tobias B., 20246 Hamburg (DE); LU, Shun, 20246 Hamburg (DE); KÖRBELIN, Jakob, 20246 Hamburg (DE); WU, Guochao, 20246 Hamburg (DE); LIU, Shuya, 20246 Hamburg (DE)
(74) Representative: Stüven, Ralf

(57) **Abstract**

The present invention relates to a recombinant vector for targeting to kidney cells comprising a targeting peptide. Also, a nucleic acid encoding a recombinant adeno associated virus (AAV) capsid and a recombinant adeno associated capsid protein comprising the targeting peptide are contemplated. The invention moreover refers to recombinant vectors for use as a medicament and for the use in the treatment of kidney diseases.

## Description

### FIELD OF THE INVENTION

The present invention relates to a recombinant vector for targeting kidney cells, comprising a targeting peptide comprising the sequence QVLXXXX. Also, a nucleic acid encoding a recombinant adeno associated virus (AAV) capsid and a recombinant AAV capsid protein comprising the targeting peptide are contemplated. The invention moreover refers to recombinant vectors for use as a medicament, for the use in the treatment of kidney diseases.

### BACKGROUND OF THE INVENTION

Gene therapy is a promising treatment option that uses therapeutic genes to treat or prevent diseases. Several gene therapy approaches are currently tested, including replacement of mutated genes that cause diseases by healthy non-mutated copies of the respective gene (gene replacement), inactivation of mutated genes that function improperly (gene silencing), or correcting gene mutations in somatic cells (gene editing).

In the field of gene therapy, the specificity of recombinant vectors used to deliver therapeutic genes plays an important role. Non-specific vectors with broad tropism a prone to induce a large number of different cell types, which on the one hand leads to decreased efficiency, as only a small number of the actual target cells is reached, and on the other hand, the transduction of non-target cells can lead to undesirable side effects. Particularly, kidney-targeted gene therapy is difficult because the kidney has a complex anatomical structure composed of various cell types in different renal compartments.

AAVs as vectors are promising candidates for use in clinical practice because they are considered to be relatively safe. From the clinical perspective, AAV vectors have been successfully applied to gene therapy in tissues such as the liver and retina, while little progresses in the kidney have been made. Many kidney diseases, especially the glomerular diseases, are caused by single-gene defects (Hildebrandt (2010)). However, kidney-targeted gene therapy is particularly difficult, because the kidney has a complex anatomical structure composed of various cell types in different renal compartments. Furthermore, previous studies reported that AAV2 and other AAV serotypes were not able to target kidney for gene therapy due to insufficient cell-specificity and inefficient transduction (Rubin et al. (2020)). It is therefore necessary to broaden the tropism of AAV and to use well-designed strategies for in vivo selection to identify viral vectors with special capsid surface that can target clinically relevant cell types in the kidney.

Endothelial cells are present ubiquitously, but they have different characteristics and functions even within one individual organ. In respect of cellular functions, endothelial cells in the glomerulus are different from the ones located in other parts of the kidney. They form the essential layer of the glomerular filtration barrier, thus play a critical role in glomerular diseases. However, there are no tools to specifically target glomerular endothelial cells so far.

Thus, there is a need to improve recombinant vectors for gene therapy, in particular to improve the cell specificity of recombinant vectors. Especially there is a need to improve the targeting of kidney cells and in particular the targeting of glomerular endothelial cells.

### OBJECTIVES AND SUMMARY OF THE INVENTION

The present invention solves the above-mentioned problem by using a recombinant vector for the targeted gene transfer into kidney cells. In particular, the invention relates to a novel peptide, which specifically binds kidney cells.

Accordingly, a first aspect of the invention refers to a recombinant vector for targeting kidney cells, comprising a targeting peptide comprising the sequence QVLXXX, wherein each X at position 4 to 6 is independently selected from any naturally occurring amino acid.

Such a recombinant vector allows the specific targeting of the kidney cells. In particular, the recombinant vector enables the targeting cells of the glomerular endothelium of the kidney. More particularly, the recombinant vector as described herein enables targeted gene transfer into the glomerular endothelium in the kidney.

Therefore, such a recombinant vector enables the gene therapeutic treatment of defined diseases or conditions of the kidney.

Typically, the targeting peptide comprises at most 20 amino acids, preferably at most 10 amino acids, more preferably at most 9 amino acids and even more preferably 8 amino acids. In a specific embodiment, the targeting peptide comprises at most 7 amino acids.

In a specific embodiment, the targeting peptide comprises at most 7 amino acids. Accordingly, in one embodiment the targeting peptide has the sequence QVLXXXX, wherein each X at position 4 to 7 is independently selected from any naturally occurring amino acid.

In some embodiments, X at position 6 of the targeting peptide is R. Accordingly, in one embodiment the targeting peptide has a sequence as set out in in SEQ ID NO: 1, i.e. QVLXXR, wherein each X at position 4 to 5 is independently selected from any naturally occurring amino acid, in particular SEQ ID NO: 2, i.e. QVLXXRX, wherein each X at position 4 to 5 and 7 is independently selected from any naturally occurring amino acid.

The targeting peptides as disclosed herein convey a high transduction specificity and efficiency for targeting a recombinant vector to kidney tissue, in particular for targeting cells of the glomerular endothelium of the kidney.

In yet another embodiment, the targeting peptide comprises the sequence QVLVYRE (SEQ NO.: 3).

Typically, the targeting peptide is exposed to the surface of the recombinant vector. The exposure of the targeting sequence on the surface of the vector increases the specificity of the vector for the targeted cells.

In another embodiment, the vector is an adeno associated virus (AAV) vector.

AAVs are promising candidates for use in clinical practice because they are considered as relatively safe. AAV vectors are capable of introducing a transgene into a cell or tissue where it is expressed stable, leading to a long-lasting and efficient expression. At the same time, these vectors do not possess any known pathogenic mechanism (Tenenbaum et al. (2003)). Therefore, they are particularly suitable for treating human kidney diseases.

In a particular embodiment, the AAV is serotype AAV2.

Typically, the targeting peptide is incorporated into at least one capsid protein of the recombinant vector.

In some embodiments, the targeting peptide is incorporated into each capsid protein of the recombinant vector.

An incorporation of the targeting peptide in the capsid protein conveys the specificity of the recombinant vector to the target cell. Therefore, an incorporation of the targeting peptide into each capsid protein of the recombinant vector is advantageous due to its increased specific binding for the targeted kidney cells.

In a particular embodiment, the recombinant vector contains 60 copies of the targeting peptide.

In a specific embodiment the targeting peptide is introduced into the capsid protein after an amino acid residue corresponding to R588 of (virion protein 1) VP1 of AAV2.

Another embodiment refers to a recombinant vector as described herein, wherein the recombinant vector comprises a transgene.

Such a transgene, being packed in the capsid may be transferred by the recombinant vector to the targeted cell where transcription and/or translation of the transgene may occur. For example, a mutated gene, leading to an altered function or malfunctioning of the targeted cell may be replaced by non-mutated copies of the mutated gene in the targeted cell (gene replacement), a gene in the targeted cell may be inactivated (gene silencing) or a gene in somatic cells may be corrected (gene editing). Therefore, a transgene being packed in the capsid can help to treat/cure diseases caused by gene mutations of kidney cells.

Accordingly, another aspect of the invention refers to the recombinant vector as described herein for use as a medicament.

Yet another aspect of the invention refers to the recombinant vector as described herein use in the prevention/treatment of a condition selected from the group of kidney diseases, glomerular diseases, disorders related to the renal glomeruli.

One embodiment refers to the recombinant vector for the use as described above, wherein the condition is glomerulonephritis.

Another embodiment refers to the recombinant vector for the use as described above, wherein the cells of the glomerular endothelium of the kidney are specifically transduced.

A further aspect of the invention refers to a recombinant AAV capsid protein comprising the targeting peptide as described herein.

Another aspect of the invention refers to a nucleic acid encoding the recombinant AAV capsid protein as described herein.

### FIGURE LEGENDS

**Figure 1****. Selection of AAV2 heptamer peptide library in** vivo
   Screening of AAV2 mutant with peptide insertion site at amino acid position R588 (VP1 numbering) using an *in vivo* selection protocol (Korbelin et al. (2017)) being adapted for the selection in murine kidney
**Figure 2****. Fluorescence microscopy of transduced murine kidney cells, expressing GFP**
   Fluorescence microscopy of murine kidney cells. AAV2-GEC loaded with GFP cDNA intravenously administrated in mice to visualize transduced cells. Subsequent immunofluorescence microscopy shows that glomerular cells are transduced in the kidney by AAV2-GEC, but not by AAV2-WT. Enlarged image on the right hand side.

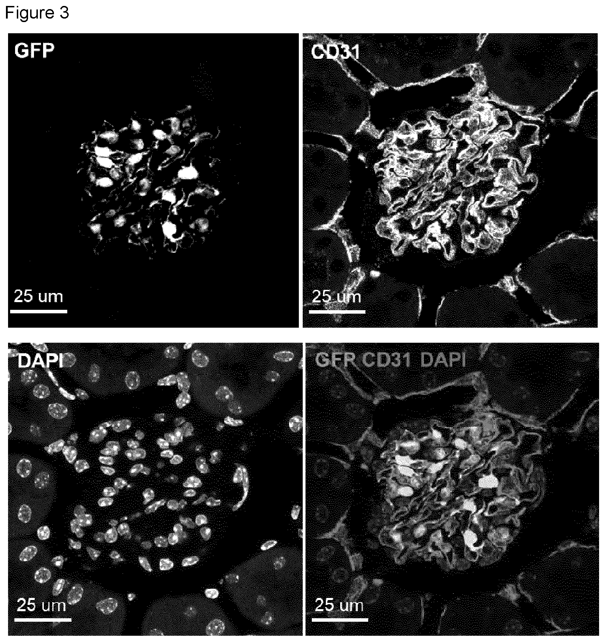

**Figure 3****. Fluorescence microscopy of AAV2-GEC transduced murine kidney cells, expressing GFP and co-stained with CD31**
   Fluorescence microscopy of murine kidney endothelial cells transduced with AAV2-GEC. AAV2-GEC loaded with GFP cDNA intravenously administrated in mice. GFP is co-stained with endothelial cell marker CD31 in the glomerulus, indicating that AAV2-GEC specifically targets glomerular endothelial cells in the kidney.

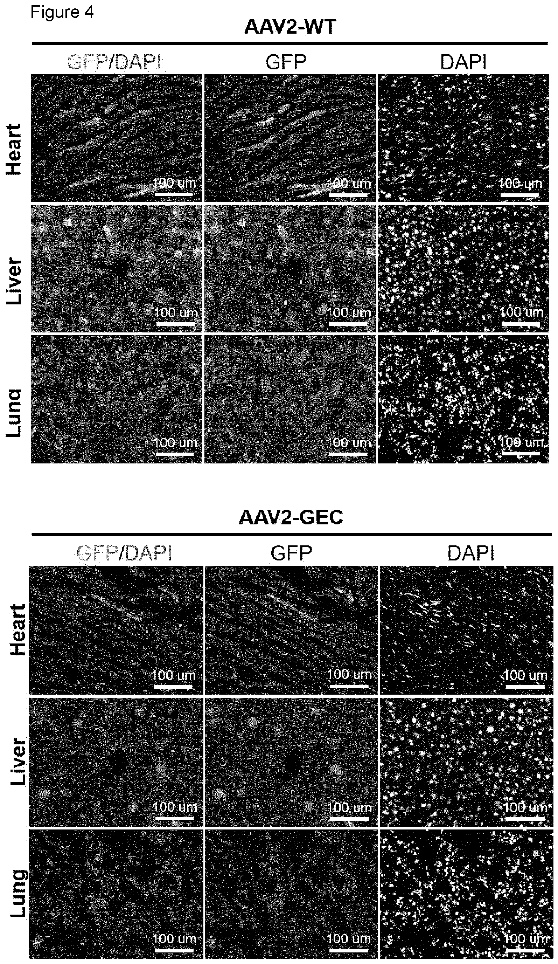

**Figure 4****. Fluorescence microscopy of transduced murine heart, liver and lung cells**
   GFP delivered by AAV2-GEC exhibits no obvious signal in the heart, liver or lung, which are regarded as off-targets, but GFP delivered by AAV2-WT shows strong signals in these organs. Cell nuclei are counterstained with DAPI.

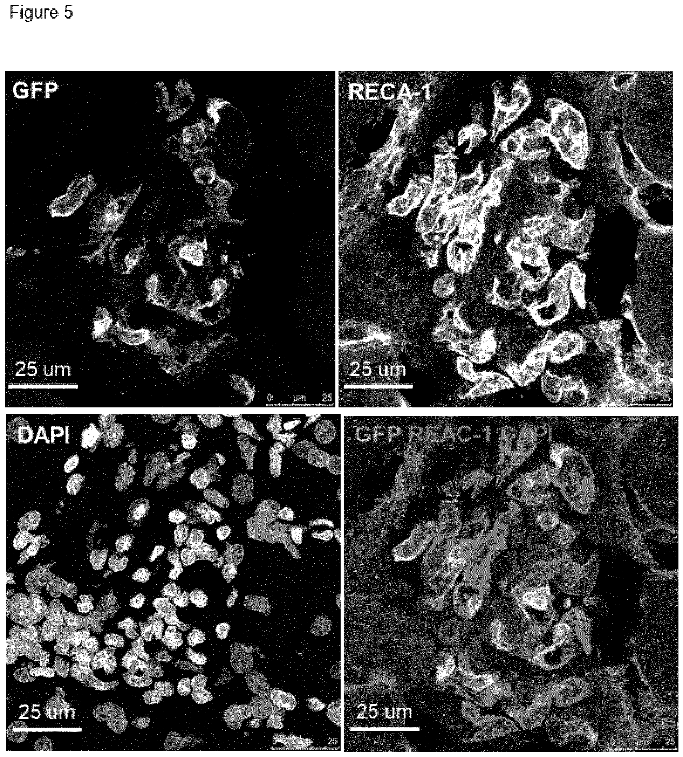

**Figure 5****. Fluorescence microscopy of AAV2-GEC transduced rat kidney cells, expressing GFP and co-stained with RECA-1**
   AAV2-GEC loaded with GFP cDNA intravenously administrated in rats. Co-staining of GFP with endothelial cell marker RECA-1 in the glomerulus, indicates that AAV2-GEC specifically targets glomerular endothelial cells in the rat kidney.

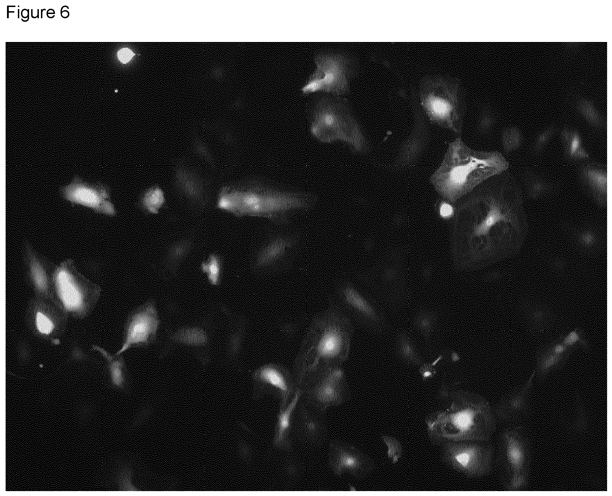

**Figure 6****. Fluorescence microscopy of transduced human endothelial cells.**
   Primary human glomerular endothelial cells transduced with AAV2-GEC loaded with GFP cDNA.

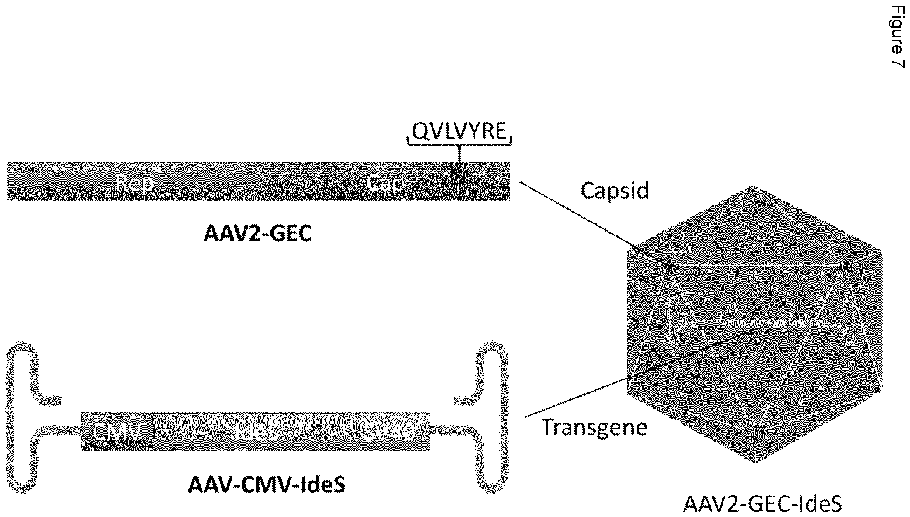

**Figure 7****. Schematic of the AAV2-GEC+IdeS construct used to treat experimental glomerulonephritis**
   Specific delivery of IdeS DNA under the CMV promotor to the glomerular endothelium by AAV2-GEC to efficiently cleave circulating IgG. IdeS (IgG-degrading enzyme of S.pyogenes): an endopeptidase which can cleave (human) IgG. *GEC: Glomerular endothelial cells. CMV: Cytomegalovirus.*

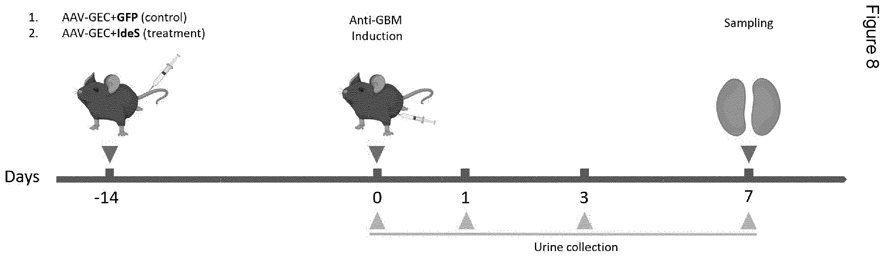

**Figure 8****. Scheme of animal models and treatment**
   Wild-type mice were divided into two groups and injected with AAV2-GEC packaging GFP and IdeS DNAs. 14 days later, both groups were injected with anti-GBM antibody to induce the glomerulonephritis. Urine was collected on day 0, 1, 3 and 7 after anti-GBM injection and kidney was collected on day 7. GBM: Glomerular basement membrane.

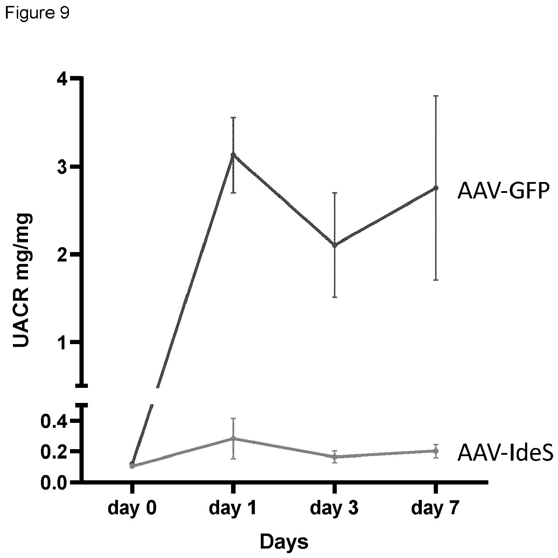

**Figure 9****. Efficacy of IdeS in experimental glomerulanephritis mice**
   Urine albumin-creatinine ratio (UACR) over time in AAV-IdeS treated and in AAV-GFP treated control mice after injection of anti-GBM serum. The UACR of control mice increased nearly 10-fold after anti-GBM injection, while the UACR of Ides-treated mice remained at baseline levels. Data shown are means ± SEM (n = 10 for each group).

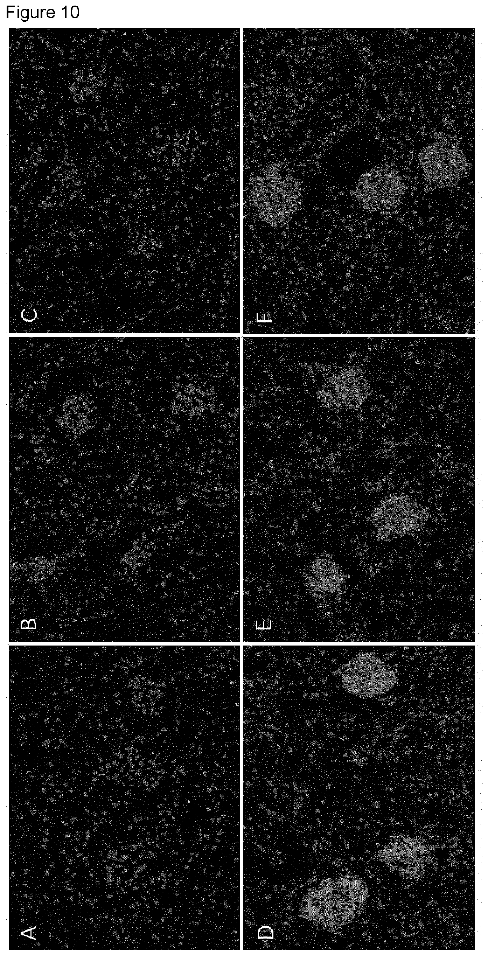

**Figure 10****. Accumulation of sheep-IgG on GBM**
   AAV2-GEC+IdeS cleaved the sheep-IgG, therefore no obvious accumulation of sheep-IgG was detected after treatment. Remaining intact sheep anti-GBM IgG was detected using AL555-conjugated donkey anti-sheep IgG antibody in the kidneys of mice from the AAV-IdeS treated group (A-C) and from mice of the AAV-GFP treated control group (D-F).

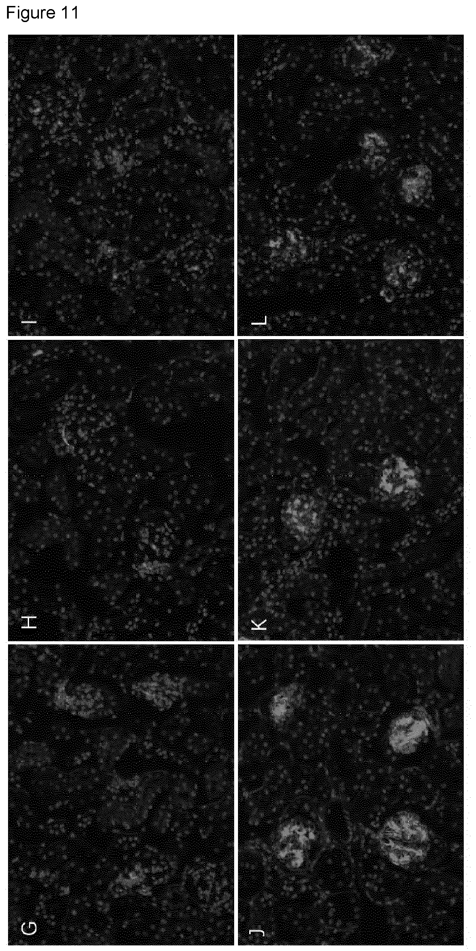

**Figure 11****. Accumulation of mouse-IgG on GBM**
   Accumulation of mouse-IgGs on the GBM was prominently prevented due to the efficient cleavage of Sheep-IgG by IdeS. Deposition of mouse IgG in the kidneys was detected using Cy3-conjugated donkey anti-mouse IgG antibody. (G-I) show samples from the AAV-IdeS treated group, while (J-L) represent the AAV-GFP treated control group.

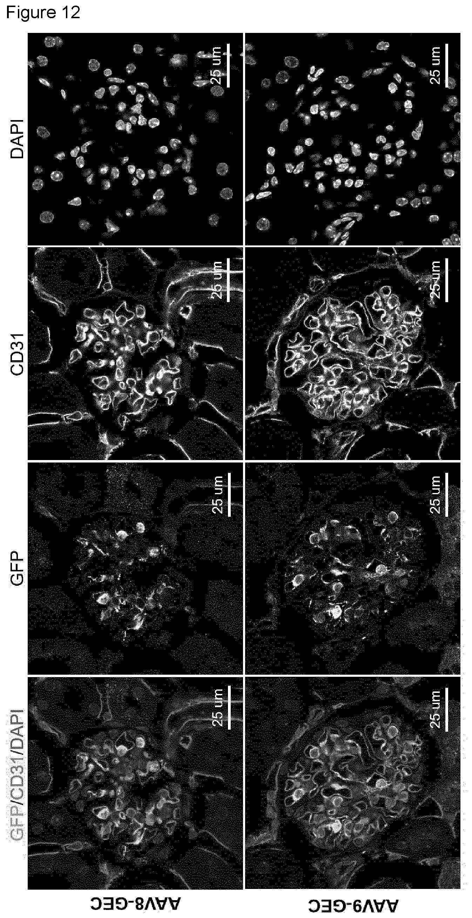

**Figure 12****. Fluorescence microscopy of AAV8-GEC and AAV9-GEC transduced murine kidney cells, expressing GFP and co-stained with CD31**
   Fluorescence microscopy of murine kidney endothelial cells transduced with AAV8-GEC or AAV9-GEC. AAV8-GEC or AAV9-GEC loaded with GFP cDNA was intravenously administrated in mice. GFP is co-stained with the endothelial cell marker CD31 in the glomerulus, indicating that GEC peptide motif (QVLVYRE) in other AAV serotypes also specifically targets glomerular endothelial cells in the kidney.

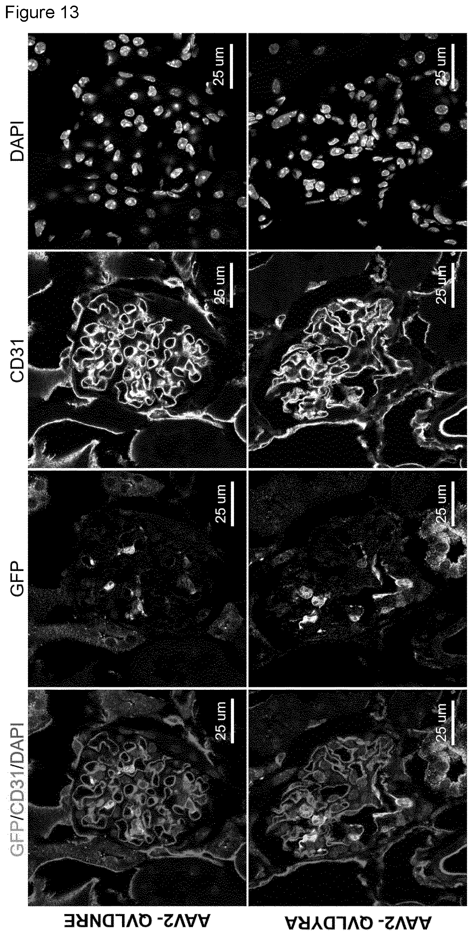

**Figure 13****. Fluorescence microscopy of AAV2-QVLDNRE and AAV2-QVLDYRA transduced mouse kidney cells, expressing GFP and co-stained with CD31**
   Fluorescence microscopy of murine kidney endothelial cells transduced with AAV2-QVLDNRE or AAV2-QVLDYRA. AAV2-QVLDNRE or AAV2-QVLDYRA loaded with GFP cDNA was intravenously injected in mice. GFP is co-stained with the endothelial cell marker CD31 in the glomerulus, indicating that peptide motif (QVLXXRX) specifically targets glomerular endothelial cells in the kidney.

### DETAILED DESCRIPTION OF THE INVENTION

The present invention as illustratively described in the following may suitably be practiced in the absence of any element or elements, limitation or limitations, not specifically disclosed herein.

The present invention will be described with respect to particular embodiments and with reference to certain figures but the invention is not limited thereto but only by the claims.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense. If a specific meaning is conveyed to certain terms, definitions of terms will be given in the following in the context of which the terms are used.

The invention thus relates in a first aspect to a recombinant vector for targeting kidney cells, comprising a targeting peptide comprising the sequence QVLXXX, wherein each X at position 4 to 6 is independently selected from any naturally occurring amino acid.

A "vector" is any molecule or moiety suitable to be used in gene therapy that is capable to transfer polynucleotides to a targeted cell.

A vector according to the invention comprises a targeting peptide, which mediates the cell specificity of the vector for a targeted cell. Exemplary, a vector according to the invention may be a lipid nanoparticle, a virus, in preferably a retrovirus, an adenovirus or adeno-associated virus (AAV), more preferably an AAV.

Where the term "recombinant vector" is used, it indicates that the vector has been modified by the introduction of a heterologous nucleic acid or protein or by an alteration of a native nucleic acid or protein. Thus, a "recombinant vector" according to the invention is any vector as described above which is derived from the expression of genetically modified polynucleotides and/or which comprises genetically modified nucleotides or heterologous or modified proteins.

The term "transduction" as used herein refers to the process of introducing nucleic acids into mammalian cells by viral methods.

The term "targeting" refers to the specific direction of the recombinant vector as disclosed herein to a specific type of cell.

The delivery of genetic material to a target cell or tissue by means of recombinant vectors is known by the person skilled in the art and may be performed in a living organism (*in vivo*) or in cell culture (*in vitro*)*.*

The term "tissue" as used herein refers to an ensemble of similar cells and their extracellular matrix from the same origin that together carry out a specific function of an organ. Therefore, the present invention also relates to a recombinant vector for targeting to kidney tissue.

The term "targeting peptide" relates to a peptide being displayed on the recombinant vector, which mediates the cell specificity of the recombinant vector for a targeted cell by binding to the respective targeted cell. In particular, the targeting peptide according to the invention specifically binds to kidney cells.

A further embodiment refers to a recombinant vector for targeting kidney cells, comprising a targeting peptide comprising the sequence QVLXXX, wherein each X at position 4 to 6 is independently selected from any naturally occurring amino acid, and wherein the recombinant vector binds specifically to the kidney cells.

A further embodiment refers to a recombinant vector for targeting kidney cells, comprising a targeting peptide comprising the sequence QVLXXX, wherein each X at position 4 to 6 is independently selected from any naturally occurring amino acid, and wherein the recombinant vector binds specifically to the glomerular endothelium of the kidney.

Endothelial cells form a single cell layer that lines all blood vessels and regulates exchanges between the bloodstream and the surrounding tissues. They are present ubiquitously, but they have different characteristics and functions even within one individual organ. In respect of cellular functions, endothelial cells in the glomerulus are different from the ones located in other parts of the kidney. They form the essential layer of the glomerular filtration barrier and thus play a critical role in glomerular diseases.

Another embodiment refers to the recombinant vector as described herein, wherein the targeting peptide comprises at most 20 amino acids, preferably at most 10 amino acids, more preferably at most 9 amino acids, and even more preferably 8 amino acids.

Typically, the targeting peptide comprises at most 7 amino acids. Accordingly, in one embodiment, the targeting peptide comprising the sequence QVLXXXX, wherein each X at position 4 to 7 is independently selected from any naturally occurring amino acid.

Some embodiments refer to the recombinant vector as described herein, where X at position 6 of the sequence of the targeting peptide is R. Accordingly, in one embodiment the targeting peptide may comprise the sequence as set out in SEQ ID NO: 1 (QVLXXR).

In a specific embodiment the targeting peptide may comprise the sequence as set out in SEQ ID NO: 2 (QVLXXRX). Exemplary targeting peptides are set out in sequences SEQ ID NO: 3 to 5.

A further embodiment refers to the recombinant vector as described herein, wherein the targeting peptide comprises the sequence QVLVYRE. Thus, in some embodiments the targeting peptide may comprise the sequence as set out in SEQ ID NO: 3.

In order to evaluate the cell specify of a targeting peptide for the targeted kidney cells a method as follows can be used, comprising the following steps:
a) providing a recombinant vector for targeting to kidney cells, comprising a targeting peptide;
b) loading the recombinant vector of step a) with a gene encoding for a reporter protein;
c) contacting the recombinant vector b) with kidney cells (target cells) and cells of at least one cell type being different from the kidney cells (control cells);
d) analyzing the cells of step c) for a signal derived from the expression of the reporter gene, and
e) comparing a signal intensity of the signal obtained in step d) derived from the target cells with a signal intensity of control cells, wherein an increase of the signal in the target cells versus the control cells indicates that the targeting peptide is cell specific for kidney cells.

The reporter gene may for example be a gene encoding GFP or DsRed or luciferase. The analysis of the cells for the respective signal of the expressed reporter gene may be accomplished by fluorescence microscopy.

Another embodiment refers to the recombinant vector as described herein, wherein the targeting peptide is exposed to the surface of the recombinant vector.

Some embodiments refer to the recombinant vector as described herein, wherein the recombinant vector is a viral vector.

Viral vectors are tools commonly used to deliver genetic material into targeted cells. Exemplary, but not limiting a viral vector may be a retrovirus, a lentivirus, an adenovirus or an adeno-associated virus.

Yet another embodiment refers to the recombinant vector as described herein, wherein the recombinant vector is an adeno associated virus (AAV) vector.

AAV are small non-enveloped viruses, which belong to the family of Parvoviridae and which comprise of a capsid with 60 proteins being arranged in a T = 1 icosahedral symmetry. The AAV genome consists of single stranded DNA of about 4.7 kilo bases. It comprises T-shaped inverted terminal repeats (ITRs) of 145 bases on each end and carries two open reading frames (ORF): rep and cap, which encode for functional and capsid proteins. The ORF rep encodes for four Rep proteins (Rep78, Rep68, Rep52 and Rep40). Rep 78 and Rep 68 have site-specific, single-strand endonuclease, DNA helicase, and ATP activities, respectively, which are responsible for DNA replication. Rep52 and Rep40 are responsible for packaging of DNA flanked by ITRs into capsids. The ORF cap encodes for three capsid proteins: VP1, VP2 and VP3. Additionally, the VP1 mRNA encodes for the assembly-activating protein (AAP) in a different reading frame. Based in the differences in the amino acid sequences of the capsid proteins, different AAV serotypes are distinguished, i.e. AAV1, AAV2, AAV3, AAV4, AAV5, AAV6, AAV7, AAV8, AAV9, AAV10, AAV11, AAV12, AAVrh10. The data presented herein show that the targeting peptide can be used in different AAV variants. Thus, in a specific embodiment the AAV is selected from the group consisting of AAV1, AAV2, AAV3, AAV4, AAV5, AAV6, AAV7, AAV8, AAV9, AAV10, AAV11, AAV12, AAVrh10 or variants thereof or a combination thereof. In one embodiment the AAV is selected from the group consisting of AAV2, AAV8 and AAV9.

Typically, the serotype of the AAV is AAV2. AAV vectors of serotype 2 (AAV2) are of particular importance for clinical use.

A serotype corresponds to a variant subspecies of AAV, which owing to its profile of expression of capsid surface antigens has a distinctive reactivity, which can be used to distinguish it from other variant subspecies. Typically, a virus having a particular AAV serotype does not efficiently cross-react with neutralizing antibodies specific for any other AAV serotype.

One embodiment refers to an AAV comprising a targeting peptide having the sequence QVLXXX. A more specific embodiment refers to an AAV comprising a targeting peptide having the sequence QVLXXXX. More specific embodiments refer to an AAV comprising a targeting peptide having the sequence as set out in SEQ ID NO: 1, in particular in having a targeting peptide having the sequence as set out in SEQ ID NO: 2. Exemplary embodiments refer to an AAV comprising a targeting peptide having any one of the sequences SEQ ID NOs: 3 to 5.

A specific embodiment refers to an AAV comprising a targeting peptide having the sequence set out in SEQ ID NO: 3.

Exemplary embodiments refer to an AAV comprising a targeting peptide having any one of the sequences SEQ ID NOs: 3 to 5.

One embodiment refers to an AAV2 comprising a targeting peptide having the sequence QVLXXX. A more specific embodiment refers to an AAV2 comprising a targeting peptide having the sequence QVLXXXX. More specific embodiments refer to an AAV2 comprising a targeting peptide having the sequence as set out in SEQ ID NO: 1, in particular in having a targeting peptide having the sequence as set out in SEQ ID NO: 2. Exemplary embodiments refer to an AAV2 comprising a targeting peptide having any one of the sequences SEQ ID NOs: 3 to 5.

A specific embodiment refers to an AAV2 comprising a targeting peptide having the sequence set out in SEQ ID NO: 3.

In another embodiment of the recombinant vector, the targeting peptide is incorporated into at least one capsid protein of the recombinant vector.

For example, an AAV capsid is composed of three viral proteins (VPs): VP1, VP2, and VP3. The VP composition of the AAV capsid is estimated to be a molar ratio of 1:1:10, indicating that one AAV capsid likely has 5 VP1, 5 VP2, and 50 VP3. Therefore, in one embodiment the targeting peptide may be incorporated in the capsid proteins VP1 or VP2 or VP3 of an AAV vector.

In another embodiment, the targeting peptide is incorporated in each of the capsid proteins VP1 and VP2 and VP3.

Thus, in a particular embodiment, the targeting peptide of the recombinant vector is incorporated into each capsid protein of the recombinant vector.

Typically, the recombinant vector contains 60 copies of the targeting peptide. As an AAV contains 60 capsid proteins, in such embodiment the recombinant vector contains 60 copies of the targeting peptide.

In some embodiments the targeting peptide is introduced into the capsid protein after an amino acid residue corresponding to R588 of (virion protein 1) VP1 of AAV2.

Some embodiments refer to chimeric AAVs, in which the capsid proteins stem from different AAV serotypes (e.g. VP1 stemming from AAV and VP2 und VP3 stemming from AAV2), or AAVs in which all capsid proteins contain sequence sections stemming from different AAV serotypes.

One embodiment refers to a recombinant AAV capsid protein comprising a targeting peptide having the sequence QVLXXX. A more specific embodiment refers to a recombinant AAV capsid protein comprising a targeting peptide having the sequence QVLXXXX.

More specific embodiments refer to a recombinant AAV capsid protein comprising a targeting peptide having the sequence as set out in SEQ ID NO: 1, in particular in having a targeting peptide having the sequence as set out in SEQ ID NO: 2. Exemplary embodiments refer to a recombinant AAV capsid protein comprising a targeting peptide having any one of the sequences SEQ ID NOs: 3 to 5.

A specific embodiment refers to a recombinant AAV capsid protein comprising a targeting peptide having the sequence set out in SEQ ID NO: 3. One embodiment refers to a recombinant AAV2 capsid protein comprising a targeting peptide having the sequence QVLXXX. A more specific embodiment refers to a recombinant AAV2 capsid protein comprising a targeting peptide having the sequence QVLXXXX.

More specific embodiments refer to a recombinant AAV2 capsid protein comprising a targeting peptide having the sequence as set out in SEQ ID NO: 1, in particular in having a targeting peptide having the sequence as set out in SEQ ID NO: 2. Exemplary embodiments refer to a recombinant AAV2 capsid protein comprising a targeting peptide having any one of the sequences SEQ ID NOs: 3 to 5.

A specific embodiment refers to a recombinant AAV2 capsid protein comprising a targeting peptide having the sequence set out in SEQ ID NO: 3. The recombinant vector of the invention may be used for cell specific delivering of at least one transgene. Accordingly, on embodiment refers to a recombinant vector comprising a transgene. Thus, some embodiments refer to an AAV comprising a transgene. In other words, the transgene is packaged within the capsid.

Typically, the transgene useful in the present invention will be a nucleotide sequence encoding a polypeptide of interest. The polypeptide may be an enzyme, ion channel, receptor (e.g., a GPCR), hormone, cytokine, chemokine, or an antibody or antibody fragment. Included within the scope of this invention is the insertion of one, two, or more transgenes.

In a specific embodiment, the transgene is a therapeutically active prophylactic active transgene.

As used herein, the term "therapeutically active" refers to any agent which can be used in treating, managing, or ameliorating symptoms associated with a disease or disorder, where the disease or disorder is associated with a function to be provided by a transgene. As used herein, the term "prophylactically active" refers to any agent which can be used in the prevention, delay, or slowing down of the progression of a disease or disorder, where the disease or disorder is associated with a function to be provided by a transgene.

In a specific embodiment the transgene comprises a reporter gene such as the cDNA of green fluorescent protein (GFP). After transduction of the reporter gene by the virus to the target cell, the reporter gene may be expressed in the target cell, enabling an identification of the successfully transduced cell.

Accordingly, a further aspect of the invention refers to a method of delivering a transgene to a cell, said method comprising contacting said cell with the recombinant vector, e.g. an AAV as described herein.

Another aspect refers to a pharmaceutical composition for use in delivering a transgene to a target tissue of a subject in need thereof, said pharmaceutical composition comprising the recombinant vector, e.g. an AAV, comprising the targeting peptide as described herein.

Accordingly, the invention also refers to a pharmaceutical composition for use in delivering a transgene to a target tissue of a subject in need thereof, said pharmaceutical composition comprising a recombinant AAV capsid protein, comprising the targeting peptide as described herein.

A further aspect of the invention refers to a recombinant vector as described herein, for use as a medicament.

Another aspect of the invention refers to the recombinant vector as described herein for use in the prevention/treatment of a condition selected from the group of kidney diseases, glomerular diseases, disorders related to the renal glomeruli.

Glomerular diseases damage the glomeruli resulting in a leakage of protein and sometimes red blood cells into the urine. The glomeruli are a tiny network of blood vessels that filter waste and remove extra fluids from the blood. When glomeruli are damaged and do not function as they should, it is called glomerular disease. Sometimes a glomerular disease interferes with the clearance of waste products by the kidney, so they begin to build up in the blood.

One embodiment refers to the use of the recombinant vector as described herein, wherein the recombinant vector is used for the prevention/treatment of glomerulonephritis.

Glomerulonephritis is a type of inflammatory disease of the kidney characterized by inflamed glomeruli and is caused by certain antibody-antigen complexes in the blood filtering through the kidney.

Another embodiment refers to the use of the recombinant vector as described herein, wherein the cells of the glomerular endothelium of the kidney are specifically transduced.

Another aspect of the invention refers to a recombinant AAV capsid protein comprising the targeting peptide as described herein.

Yet another aspect of the invention refers to a nucleic acid encoding the recombinant AAV capsid protein as described herein.

### Experiments

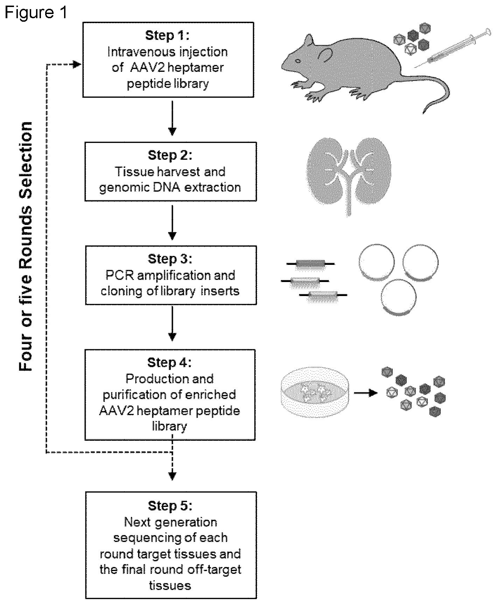

In the present invention, an AAV2 heptamer peptide library with peptide insertion site at amino acid position R588 (VP1 numbering) was screened using an in vivo selection protocol (Korbelin et al. 2017), which was adapted for the selection in the kidney (Figure 1).

A heptamer having particular specificity for the glomerular endothelial cells was peptide. Based on this sequence, the recombinant viral vector rAAV2-QVLVYRE (hereafter referred to as AAV2-GEC) was produced, which expresses the peptide sequence QVLVYRE as a partial sequence of each virion protein (VP), resulting in a total of 60 peptide copies in the fully assembled viral capsids. Subsequently, vector AAV2-GEC was administered intravenously (via tail vein) to mice and a clear specificity of the vector for the glomerular endothelial cells was demonstrated in the kidney in vivo.

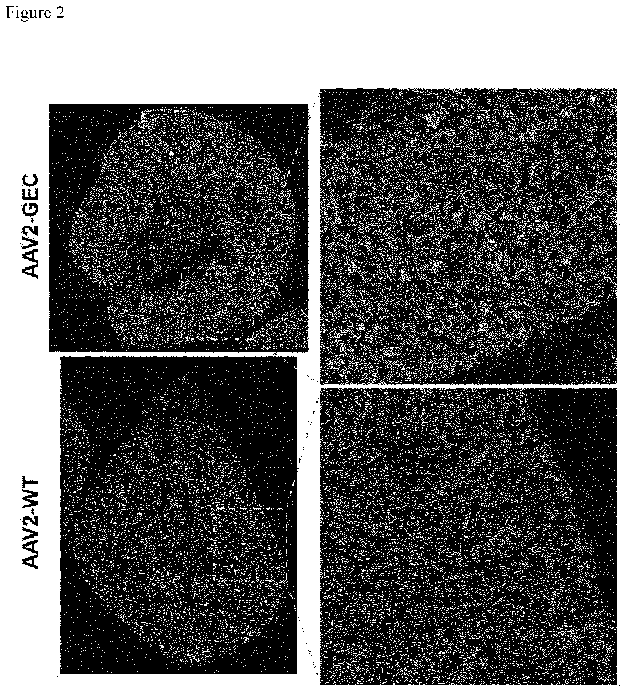

AAV2-GEC loaded with GFP cDNA was intravenously administrated in mice to visualize transduced cells. Subsequent immunofluorescence microscopy showed that glomerular endothelial cells were transduced in the kidney by AAV2-GEC, but not AAV2-WT (wildtype) (Figure 2). The specificity was confirmed by co-staining with CD31, which is a marker for endothelial cells (Figure 3). GFP delivered by AAV2-GEC exhibited no obvious signal in the rest of tissues or organs, which are regarded as off-targets, but GFP delivered by AAV2-WT showed strong signals in these tissues and organs (Figure 4).

The target transduction efficiency of AAV2-GEC was evaluated in rats. AAV2-GEC loaded with GFP cDNA was intravenously administrated in rats to visualize transduced cells. Subsequent immunofluorescence microscopy of the kidney showed that glomerular endothelial cells were transduced by AAV2-GEC (Figure 5), suggesting high transduction efficiency of AAV2-GEC in rat glomerular endothelial cells.

The target transduction efficiency of AAV2-GEC was evaluated in primary human glomerular endothelial cells. The cells were transduced with AAV2-GEC loaded with GFP cDNA. Four days after transduction, the highest GFP signal was detected in cells (Figure 6), suggesting high transduction efficiency of AAV2-GEC in human glomerular endothelial cells.

The peptide QVLVYRE was subcloned into the AAV serotype 8 or 9. AAV8-GEC or AAV9-GEC loaded with cDNA of green fluorescent protein (GFP) was intravenously administrated in mice to visualize transduced cells. Subsequent fluorescence microscopy showed an equal targeting specificity compared to AAV2-GEC **(****Figure 12****),** suggesting that targeting specificity mediated by the peptide QVLVYRE may be widespread in combination with other AAV serotypes.

Peptide QVLDNRE, QVLDYRA, QVLVYLE and QVLGYRK which were enriched in the on-target tissue and sharing the same motif QVL, was cloned in AAV serotype 2. Recombinant vector loaded with cDNA of green fluorescent protein (GFP) was intravenously administrated in mice to visualize transduced cells. Subsequent fluorescence microscopy showed AAV2-QVLDNRE and AAV2-QVLDYRA having an equal targeting specificity compared to AAV2-GEC **(****Figure 13****),** indicating that the motifs with the sequences QVLXXXX and especially QVLXXRX are of importance for the targeting specificity of the recombinant vector.

Taken together, these results suggest that AAV2-GEC can specifically and efficiently transduce the glomerular endothelium in the kidney in mice, rats and potentially in human. Thus, this viral vector could be administered in a therapeutically effective amount to the patient. For instance, in an amount sufficient to improve the dysfunction or disease related or caused by renal glomeruli, or to prevent the progression of the disease.

Rapidly progressive glomerulonephritis (RPGN) is a syndrome of rapid loss of function in the kidney. It is characterized by the presence of autoantibodies at the glomerular basement membrane (GBM). IdeS (immunoglobulin G-degrading enzyme of S. pyogenes) cleaves (human) IgG antibodies and is used in the clinic for antibody-mediated diseases and autoimmune disorders including anti-GBM disease, which is a subtype of RPGN (Collin et al. (2017)). In the case of refractory anti-GBM nephritis, a single dose of IdeS administration led to rapid clearance of circulating anti-GBM antibodies. However, after about a week all treated patients rebounded (Soveri et al. (2019)). Therefore, a GEC-targeting AAV vector specifically expressing IdeS in glomeruli can not only cleave the anti-GBM antibodies locally and perhaps more efficiently, thereby better prevent the progression of glomerulonephritis, but also provide continuous improvement.

As a proof of concept, AAV2-GEC was used to specifically deliver IdeS to the glomerular endothelium for the treatment of the anti-GBM disease (Figure 8). In the mouse model of anti-GBM disease, anti-GBM sheep-IgG was injected. The circulating antibodies were accumulated on the GBM, leading to the activation and subsequently the binding of mouse-IgG against sheep-IgG on the GBM. Consequently, it resulted in the kidney glomerular damage, representing the anti-GBM disease in human. When compared to the AAV2-GEC delivering GFP as a control, the AAV2-GEC delivering IdeS successfully prevented the glomerular filtration dysfunction (evaluated by the urinary albumin to creatinine ratio (UACR)) (Figure 9). The accumulation of sheep-IgGs on the GBM was prominently prevented, suggesting the effective expression of Ides by the glomerular endothelium (Figure 10). The accumulation of mouse-IgGs on the GBM was also prominently prevented due to the efficient cleavage of Sheep-IgG by IdeS (Figure 11), which preserved glomerular filtration function, thereby prevented the disease progression. Taken together, the successful treatment of glomerulonephritis by targeting IdeS using AAV-GEC to the kidney provides a functional evidence, suggesting that AAV-GEC has therapeutic potentials for the diseases related or caused by the renal glomeruli.

### Methods

### Screening of AAV vector for targeting the kidney

An AAV vector comprising the targeting sequence as described herein may be obtained by screening using an *in vivo* selection protocol such as Korbelin et al. (2017), adapted for the selection in the kidney. For example, an "AAV2 mutant with peptide insertion site at amino acid position R588 (VP1 numbering) can be screened using the above-mentioned *in vivo* selection protocol (Korbelin et al. (2017)), being adapted for the selection in the kidney (Figure 1). A resulting variant comprises the target peptide sequence QVLVYRE (SEQ NO.: 3) as a partial sequence of each virion protein (VP), resulting in a total of 60 peptide copies in the fully assembled viral capsids. This variant is termed AAV2-GEC herein.

### Comparison of peptide motifs

By comparing AAV2-GEC with the peptide sequences, which were enriched in the on-target tissue, the different sequences sharing the same motif were identified:
**QVL**VYRE
**QVL**DNRE
**QVL**DYRA
**QVL**VYLE
**QVL**GYRK

In summary, it is found that the several sequences (namely the sequences set out in SEQ ID Nos.: 3 to 7) shared the motif QVL, which is important for the targeting specificity. Furthermore, after in vivo validation with GFP it was found that the GFP-positive peptide sequences (SEQ ID NOs 3 to 5) shared R at position 6, which indicates their importance for the targeting specificity. Thus, the motifs with the sequences QVLXXXX and QVLXXRX (SEQID NO.:2), where X is any natural amino acid, are of importance for the targeting specify of the recombinant vector.

### Production of recombinant AAV vector

Recombinant AAV vectors were produced by co-transfection of HEK293T/17 cells with the packaging plasmid AAV Rep/Cap (wild-type capsid or capsid mutants with selected peptide), the ITR-flanked transgene plasmid, and the adenoviral helper plasmid pXX6. Three days after transfection, cell lysates were subjected to freeze-thaw cycles in PBS-MK and the vectors were purified by iodixanol gradient ultracentrifugation followed by dialysis and concentration against PBS using Amicon Ultra-15 centrifugal filter devices (Millipore). Physical particles were quantified by real-time PCR with the forward primer: 5'-GGGACTTTCCTACTTGGCA-3' and the reverse primer: 5'- GGCGGAGTTGTTACGACAT-3' directed to the CMV promoter sequence. AAV titers are expressed as viral genomes per mL (vg/mL).

### Transduction of kidney cells

Intravenous injections of AAV vector were performed in eight weeks old C57BL/6J wild-type mice and six weeks old SD-rats. AAV vectors expressing GFP reporter for tissue transduction efficiency and specificity evaluation were injected at the dose of 1E+11vg per mouse and 1E+12vg per rat. Two weeks post-injection, mice and rats were sacrificed and organs were harvested for histological analysis.

### Evaluation of cell specify

The GFP and other cell markers on frozen sections were detected using the following primary antibodies: chicken anti-GFP (Thermo Scientific); rat anti-CD31 (BD Biosciences); mouse anti-rat RECA-1 (Novus Biologicals). Alexa Fluor 488-, 555-, 594-labeled secondary antibodies were used as the case may be (Invitrogen or Thermo Fisher Scientific). Images were taken by Zeiss Apotome or Leica TCS SP5 microscope.

### AAV transduction of human primary kidney glomerular endothelial cells

Human primary glomerular endothelial cells were cultured according to the manufacturer's recommendation. The cells were seeded on the gelatin-coated 12-well plates and incubated overnight to achieve approximately 70%-80% confluence. The next day, each well of cells was infected with 5E+10vg AAV2-GEC particles. Then the cells were kept in culture for five days and GFP expression was tracked during this period via fluorescence microscopy. The images were taken on post-infection day 4.

### Animal model for treating glomerulonephritis

Eight weeks old C57BL/6 male mice (from Charles River Laboratories) were randomly assigned to two groups. One group was injected with 2E+11vg AAV-IdeS and the other group was injected with 2E+11vg AAV-GFP by an intravenous injection. 14 days after AAV injection, both groups were injected with 150 ul of anti-GBM serum (PTX-001 sheep anti-rat GBM serum, Probetex Inc.) by an intraperitoneal injection. Urine was collected before and 1, 3, 7 days after anti-GBM serum injection. Animals were sacrificed 7 days after anti-GBM serum injection.

### Fluorescence immunohistochemistry

Deposition of sheep IgG and mouse IgG was assessed with immunofluorescence analysis on frozen kidney sections. Sheep IgG was detected using an Alexa Fluor 555-conjugated donkey anti sheep IgG (Invitrogen), while mouse IgG was detected using Cy3-conjugated donkey anti-mouse IgG (Jackson ImmunoResearch). Images were taken by Zeiss Apotome with a magnification of 200x.

The invention comprises the following items:
Item 1. Recombinant vector for targeting kidney cells, comprising a targeting peptide comprising the sequence QVLXXX, wherein each X at position 4 to 6 is independently selected from any naturally occurring amino acid.
Item 2. Recombinant vector according to item 1, wherein the targeting peptide comprises at most 20 amino acids, preferably at most 10 amino acids, more preferably at most 9 amino acids and even more preferably 8 amino acids.
Item 3. Recombinant vector according to item 1, wherein the targeting peptide comprises at most at most 7 amino acids.
Item 4. Recombinant vector according to item 1 or 2, wherein X at position 6 is R.
Item 5. Recombinant vector according to any one of the preceding items, wherein the targeting peptide comprises the sequence QVLVYRE.
Item 6. Recombinant vector according to any one of the preceding items, wherein the targeting peptide is exposed to the surface of the recombinant vector.
Item 7. Recombinant vector according to any one of the preceding items, wherein the recombinant vector is an adeno associated virus (AAV) vector.
Item 8. Recombinant vector according to item 7, wherein the AAV is serotype AAV2.
Item 9. Recombinant vector according to any one of the preceding items, wherein the targeting peptide is incorporated into at least one capsid protein of the recombinant vector.
Item 10. Recombinant vector according to any one of the preceding items, wherein the targeting peptide is incorporated into each capsid protein of the recombinant vector.
Item 11. Recombinant vector according to any one of the preceding items, wherein the recombinant vector contains 60 copies of the targeting peptide.
Item 12. Recombinant vector according to any one of items 7 to 11, wherein the targeting peptide is introduced into the capsid protein after an amino acid residue corresponding to R588 of (virion protein) VP1 of AAV2.
Item 13. Recombinant vector according to any one of the preceding items, wherein the recombinant vector comprises a transgene packaged within the capsid.
Item 14. Nucleic acid encoding the recombinant vector according to any one of the preceding items.
Item 15. Recombinant vector according to item 13, for use as a medicament.
Item 16. Recombinant vector according to item 13 for use in the prevention/treatment of a condition selected from the group of kidney diseases, glomerular diseases, disorders related to the renal glomeruli.
Item 17. Recombinant vector for use according to item 15, wherein the condition is glomerulonephritis.
Item 18. Recombinant vector for use according to items 15 to 17, wherein the cells of the glomerular endothelium of the kidney are specifically transduced.
Item 19. Recombinant AAV capsid protein comprising the targeting peptide as defined in items 1 to 6.
Item 20. Nucleic acid encoding the recombinant AAV capsid protein defined in item 19.

### ABBREVIATIONS

- GFP: green fluorescent protein
- DsRed: red fluorescent protein isolated from *Discosoma*
- AAV: adeno-associated virus
- AAV2: AAV vector of serotype 2
- CD31: Cluster of Differentiation 31
- DAPI: 4',6-diamidino-2-phenylindole
- RECA-1: anti-endothelial cell antibody
- WT: wild type
- VP: virion protein
- X: any naturally occurring amino acid

### REFERENCES

Tenenbaum et al. (2003) Evaluation of risks related to the use of adeno-associated virus-based vectors. Curr Gene Ther 3, 545-565.
Hildebrandt (2010) Genetic kidney diseases. Lancet 375, 1287- 1295
Rubin et al. (2020) Improving Molecular Therapy in the Kidney. Mol Diagn Ther 24, 375-396.
Korbelin et al. (2017) How to Successfully Screen Random Adeno-Associated Virus Display Peptide Libraries In Vivo. Hum Gene Ther Methods 28, 109-123.
Collin et al. (2017) Toward Clinical use of the IgG Specific Enzymes IdeS and EndoS against Antibody-Mediated Diseases. Methods Mol Biol. 1535, 339-351
Soveri et al. (2019) The IgG-degrading enzyme of Streptococcus pyogenes causes rapid clearance of anti-glomerular basement membrane antibodies in patients with refractory anti-glomerular basement membrane disease. Kidney Int. 96, 1234-1238.

## Claims

1. Recombinant vector for targeting kidney cells, comprising a targeting peptide comprising the sequence QVLXXX, wherein each X at position 4 to 6 is independently selected from any naturally occurring amino acid.

2. Recombinant vector according to claim 1, wherein the targeting peptide comprises at most 20 amino acids, preferably at most 10 amino acids, more preferably at most 9 amino acids and even more preferably 8 amino acids.

3. Recombinant vector according to claim 1, wherein the targeting peptide comprises at most at most 7 amino acids.

4. Recombinant vector according to claim 1 or 2, wherein X at position 6 is R.

5. Recombinant vector according to any one of the preceding claims, wherein the targeting peptide comprises the sequence QVLVYRE.

6. Recombinant vector according to any one of the preceding claims, wherein the targeting peptide is exposed to the surface of the recombinant vector.

7. Recombinant vector according to any one of the preceding claims, wherein the recombinant vector is an adeno associated virus (AAV) vector, wherein optionally the AAV is serotype AAV2.

8. Recombinant vector according to any one of the preceding claims, wherein the recombinant vector comprises a transgene packaged within the capsid.

9. Nucleic acid encoding the recombinant vector according to any one of the preceding claims.

10. Recombinant vector according to claim 8, for use as a medicament.

11. Recombinant vector according to claim 8 for use in the prevention/treatment of a condition selected from the group of kidney diseases, glomerular diseases, disorders related to the renal glomeruli.

12. Recombinant vector for use according to claim 10, wherein the condition is glomerulonephritis.

13. Recombinant vector for use according to claims 10 to 12, wherein the cells of the glomerular endothelium of the kidney are specifically transduced.

14. Recombinant AAV capsid protein comprising the targeting peptide as defined in claims 1 to 6.

15. Nucleic acid encoding the recombinant AAV capsid protein defined in claim 14.
